# EUROPEAN PATENT SPECIFICATION

(11) **EP 1 140 001 B1**
(45) Date of publication and mention of the grant of the patent: **04.05.2005**
(21) Application number: 99960726.0
(22) Date of filing: 17.12.1999
(51) Int. Cl.: A61K 7/48, A61K 31/185

(54) **COMPOSITION FOR TREATMENT OF BURNS**
ZUSAMMENSETZUNG ZUR BEHANDLUNG VON VERBRENNUNGEN
COMPOSITION POUR LE TRAITEMENT DES BRULURES

(30) Priority: 18.12.1998 US 215235
(43) Date of publication of application: 10.10.2001
(73) Proprietor: Palladin HealthCare International Ltd, Toronto, Ontario M5J 2L4 (CA)
(72) Inventor: DOSCH, Michael H., Toronto, Ontario M5J 2L4 (CA); Li, Xiamomao, Toronto, Ontario M5J 2L4 (CA); OSTERMANN, Kurt, Scarborough, Ontario M1M 3P8 (CA)
(74) Representative: Jones, Helen Marjorie Meredith
(86) International application number: PCT/CA1999/001185
(87) International publication number: WO 2000/037037

(56) References cited:
- PATENT ABSTRACTS OF JAPAN vol. 013, no. 021 (C-560), 18 January 1989 (1989-01-18) & JP 63 227511 A (MASAYUKI MORISHITA), 21 September 1988 (1988-09-21)
- PATENT ABSTRACTS OF JAPAN vol. 012, no. 433 (C-543), 15 November 1988 (1988-11-15) & JP 63 159317 A (TERUMO CORP), 2 July 1988 (1988-07-02)
- PATENT ABSTRACTS OF JAPAN vol. 1999, no. 06, 31 March 1999 (1999-03-31) & JP 03 120220 A (SIEGFRIED NATTERER), 22 May 1991 (1991-05-22)

## Description

### FIELD OF THE INVENTION

The present invention is directed to compositions for treatment of burns.

### BACKGROUND OF THE INVENTION

Treatment of burn injuries continues to be a significant concern in the medical field. Burns due to skin contact with flame, hot surfaces, hot liquids etc. are among the three most common accidents, both at the workplace and at home. In North America, about 95% of all burn injuries are treated by home remedies, 2.5 million subjects seek medical advice for burns annually in the US., about 100,000 of these require hospitalization [Peate, W.F., Am. Fam. Physician, **45**:1321, 1992]. These figures do not include sunburn injury.

European figures are similar, with about 5% of burn injuries that come to medical attention admitted to hospital and a mortality rate of 0.2% [Jonsson, C.E, Ann. Chir. Gynaecol, **69**:168, 1980; Hytonen, M. *et al*., Ann. Chir. Gynaecol, **76**: 218, 1987; de Roche, R. *et al*., Burns; **20**:58, 1994]. Most of the burn injuries occur at home, most are scalding accidents with gender and age distributions varying widely by region, urbanization and education.

The psycho-social impact of burn injuries is dramatic. Over 95% of costs are incurred by severe injuries, mostly (∼ two thirds) in terms of lost working days and to a lesser extent for direct care [de Roche, R. *et al*., 1994; Khoo, A.K. *et al*., Ann. Acad. Med. Singapore, **23**:680, 1994; Hansbrough, J.F. *et al*., J. Burn Care Rehabil., **16**:377, 1995]. There are, however, essentially no published data that analyze the epidemiology and impact of burn injuries which do not reach medical attention, but which often do cause loss of working days and considerable, if transient reductions in quality of life [Miller, S.F., Am. Fam. Physician, **16**:167, 1977; Mertens, D.M. *et al*., Nurs. Clim. North Am., **32**:343, 1997].

The main immediate burn injury problems include pain and primary infection. Scarring and functional impairment characterize the intermediate term. Malignancy represent a long term risk, although this is rare for most burns except those from ultraviolet and other radiation.

Beyond the well known misery of burn pains, the objective threats, severity and prognosis of burn injuries are all functions of injury extent, i.e. surface area and depth of tissue destruction. The total tissue destruction is the sum of cells directly destroyed by heat and the much larger number of cells dying due to local tissue responses including edema, leukocytic infiltration, local mediators, apoptosis and primary infection. These elements are fundamental and similar in small as well as massive burn injuries, the latter adding systemic responses in a critical care scenario with mortality rates increasing as a quantitative function of tissue loss, infection and post-burn immunosuppression.

The principles of burn therapy have changed little over the past decades, and there is some lack of basic science and animal models for in depth analysis of the cellular and molecular events following tissue burn injury. There is long-standing consensus for rapid and aggressive intervention following burn injury, including the early use of skin grafting.

One conclusion from this recognized need for rapid intervention is that following the direct tissue destruction by the hyperthermal insult, it is local and systemic biological responses that determine much of the post-injury course. This scenario is analogous to, and may include, the development of shock syndromes which can be by themselves life threatening, independently of the nature and extent of a given injury [Cason, J.S., Prog. Pediatr. Surg., **14**:3, 1981]. Other than shock, however, the immediate interference with tissue injury responses following thermotrauma has received little attention beyond general pain relief and precaution against infection [Baxter, C.R. and Waeckerle, J.F., Ann. Emerg. Med., **17**:1305, 1988; Brofeldt, B.T. *et al*., J. Burn Care Rehabil., **10**:63, 1989].

Secondary cell death due to tissue burn injury responses involves the elements of acute phase reactants such as leukocyte extravasation and mediator release. Vasoconstriction peripheral to the injury locus but hyperemia and fluid loss within the injured area conspire to reduce oxygenation, accumulate toxic detritus, and activate complement and apoptosis pathways for pronounced secondary cell death. These events create the viscious circle characteristic of even small burn injuries and their misery [Shaw, A *et al*., Br. J. Hosp. Med., **52**:583, 1994; Arturson, G., Burns, **22**:255, 1996]. In addition, the injured tissue represents an ideal, immunologically underprivileged target site for infectious agents which can dramatically threaten the recovery process.

While the need to cover burn injuries has long been recognized, ideal solutions are still elusive, and the variety of proposed remedies is profuse, ranging from honey, tree bark and animal urine or dung to potato peels to amniotic membranes, allografts and modern plastics to name but a few.

The use of tannic acids (TA) (or tannic herb extracts) as a burn remedy had been proposed early this century [Davidson, E. C., Surg. Gynecol. Obstet., **41**, 1925] based upon ancient Chinese texts. TA is the collective name for a group of undefined substances which convert putrefiable hide or skin into imputrescible leather. TA occurs in most parts of the vegetable kingdom and is more prevalent among the higher plants, especially in the barks of trees (cited from: Hupkens P. *et al*., Burns, **21**:57, 1995). The acidity of these extracts is uncertain, as hydroxy groups from the trihydroxybenzene structure are extremely weak proton donors.

The often ill described tannic herb extracts differed in their hepatotoxicity and tendency towards secondary infection [Hupkens, P. *et al*., 1995]. TA treatment has been linked to liver necrosis in burn patients, even death: in one report 14 of 16 TA-treated patients who died from their burn trauma showed definitive evidence of serious liver necrosis (reviewed in [Hupkens, P. *et al*., 1995]). This level of toxicity is unacceptable and has lead to the disappearance of TA from medical practice, although the burn-specific therapeutic profiles associated with TA's, including their analgesic and scar-sparing effects was rather consistently reported and TA use in severe burns appears to have reduced war burn mortality rates from 35% to 12% [Erb, I.H. *et al*., Ann. Surg., **117**:234, 1943].

No pathophysiological studies or conclusions are available that identify the active principle(s) in the heterogeneous mixtures of herb tannins employed as burn remedies. The proposed mechanism of action for TA was based on the perceived significance of so called "burn toxins" (a 70 year old concept no longer acceptable) released in the wound and relied on TA's ability to absorb/precipitate these putative toxins.

The use of vinegar as an antimicrobial agent is as old as the use of alcohol, and by around 1000 AD, hand washing with vinegar was recommended in ancient medical texts from China and Arabic sources such as Shen Tua (1031-1095 AD) [Chan, E.L. *et al*., Am. J. Nephrol., **14**:295, 1994]. Vinegar is an impure organic acid and a rich source of many volatile contaminants [De, V.M. *et al*., Food Add. Contam., **4:**161,1987]. It has possible antibacterial and antiviral effects, although the mechanisms of this vinegar activity is unknown. Vinegar is approved for human non-dietary use and performs well as the main ingredient of vaginal douches although mechanisms are, again, uncertain [Brinton, L.A. *et al*., Gynecol. Oncol., **38**:49, 1990; Nyirjesy, P., *et al*., Obstet. Gynecol., **90**:50, 1997].

The effectiveness of pure acetic acid, sodium acetate and vinegar have rarely been compared [Brighenti, F. *et al*., Eur. J. Clin. Nutr., **49**:242, 1995], and never with respect to antimicrobial activity. Although sodium acetate is used in some vaginal douches, their effectiveness has not been measured [Chvapil, M. *et al*., Obstet. Gynecol., **52**:88, 1978]. While acid sensitivity of bacteria is one element of antimicrobial activity, this is insufficient to explain the antimicrobial effects of vinegar, since some common food-borne bacteria are highly sensitive to vinegar, yet they survive gastric acid exposure and cause common intestinal disease [Nishikawa, Y. *et al*., Int. J. Food Microbiol., **18**:271, 1993]. There is a considerable amount of published literature on the antimicrobial effects of vinegar [Emili, H. *et al*., 1974; Larghi, O.P. *et al*., Rev. Asoc. Argent. Microbiol., **7**:86, 1975; Fasanella, R.M., Ophthalmic. Surg., 1991; Karapinar, M. and Gonul, S.A., Int. J. Food Microbiol., **16**:261, 1992; Nishikawa, Y. *et al*., 1993; Rund, C.R., Ostomy. Wound Manage., **42**:18, 1996], observations that are finding their way into the food processing industries[Dickens, J.A. *et al*., Poult. Sci., **73**:576, 1994; Entani, E. *et al*., Kansenshogaku Zasshi, **71**:443, 1997].

While vinegar treatment of killed chicken prior to freezing was shown to significantly reduce bacterial contamination, there are no published reports on the effects of vinegar on living skin [Dickens, J.A. *et al*., 1994]. A widely employed skin application of vinegar occurs in the Pacific Rim Countries where, acting a a nematocyst inhibitor, vinegar is the recommended first aid treatment against potentially life threatening jelly fish stings [Fenner, P.J. *et al*., Med. J. Aust., **143**:550, 1985; Beadnell, C.E. *et al*., Med. J. Aust., **156**:655, 1992; Fenner, P. J. *et al*., Med. J. Aust., **158**:498, 1993].

In vitro tissue culture studies suggested that acetic acid is toxic to human fibroblasts and keratinocytes in concentrations of above 0.025% (reviewed in [Rund, C.R., 1996]). These data contrast with the frequent use of vinegar douches, which are tolerated well and over prolonged periods of intravaginal use [Nyirjesy, P. *et al*., Obstet. Gynecol., **90**:50, 1997]. The applicability of such tissue culture studies to *in vivo* tissue is therefore uncertain.

### SUMMARY OF THE INVENTION

It is an object of the present invention to provide compositions for the treatment of burns. In accordance with an aspect of the present invention there is provided a composition for treating burns comprising a therapeutically effective amount of one or more weak organic acids selected from the group comprising acetic acid, vinegar, and citric acid, in a carrier which comprises a gelling agent, wherein the pH of the composition ranges from 4.0 to 4.5.

In accordance with another aspect of the present invention there is provided the use of the novel composition in the manufacture of a medicament for use in a method for treating burns.

The present invention involves a composition for treating burns. The composition comprises a therapeutically effective amount of one or more weak organic acids compatible with human skin in a suitable carrier. The weak organic acids are selected from the group comprising acetic acid, vinegar and citric acid. The suitable carrier is an aqueous based carrier in a gel form, utilizing for instance a Carbopol™ as a gelling agent. In yet another embodiment of the invention, the composition also includes one or more other agents selected from sun blocking agents, skin moisturizing agents and herb extracts. The invention allows the treatment of skin trauma arising from thermal, chemical, light sources, wherein the method comprises applying to the affected area of skin a therapeutically effective amount the composition, preferably wherein the pH ranges between 4.1 to 4.4, most preferably 4.15 to 4.25, and optimally equals 4.2.

### BRIEF DESCRIPTIONS OF THE DRAWINGS

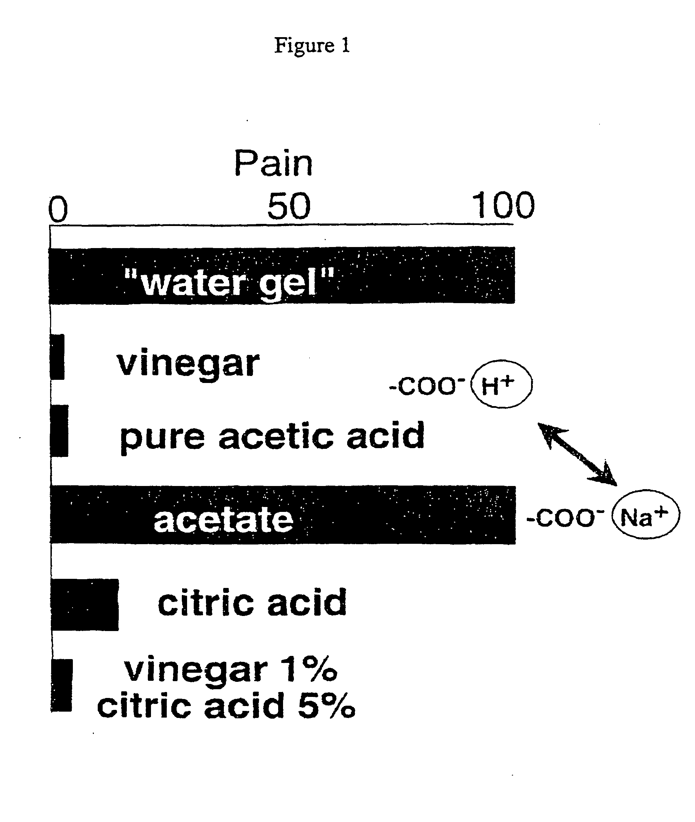

Figure 1: Illustrates data relating to patient's perception of pain, after treatment of burns with various compositions.

### DETAILED DESCRIPTION OF THE INVENTION

For the purpose of better understanding the invention, the term, burn, means an area of tissue damage, caused by excessive exposure to thermal, chemical electrical or radioactive agents. The effects may be local, resulting in cell injury or death, or both local and systemic. Burns are classified according to the depth of the tissue damage.

The present invention is directed to a composition for treating burns where the composition is effective to provide immediate interference with tissue injury responses following trauma. In contrast to topical local analgesics, which do not affect aspects of wound healing, local or systemic responses [Pedersen, J.L. *et al*., 1996], the compositions of the present invention provide both, analgesia and distinct effects on the tissue injury response which is reflected in the beneficial course of scar-less healing.

The present invention relies on the observed effectiveness of weak organic acids compatible with human skin as a burn therapeutic agent as defined by claim 1. The effectiveness of compositions of the invention has been shown using, for example, food-grade vinegar, diluted acetic acid or citric acid. Local delivery of the composition of the invention demonstrated a range of unexpected properties beneficial for the treatment of full and partial skin thickness thermal injuries due to flame, hot surface contact, scalding, as well as skin surface damage due to overexposure to sunlight.

The application of the composition of this invention is counter-intuitive, as acids can cause burns themselves when encountered in sufficient concentration. Sources of concentrated acids can cause topical bums and should, thus, be avoided. In contrast, the present invention has established that in an appropriate composition of weak acid, for example, acetic acid, vinegar and citric acid, are effective for bum therapy. These concentration/dilutions are similar to the acetic acid content of food-grade vinegar and the citric acid content of natural lemon juice, respectively.

The composition of the invention is thought to target the initial oscillations of the cycle leading to secondary cell death, thereby preventing secondary cell death with consequent improvement of the post-injury healing process. This is evidenced by the observations that although the positive impact on localized burns is dramatic, reproducible, fast, but also that the effects last beyond the 1-2 hrs of initial application.

As discussed above, the compositions of the present invention stop the collateral damage due to the cascade of local responses to thermal insult. Without the pain and consequent vasoconstriction, without extravasation and consequent edema, infiltration, mediator release and blister formation, healing can and does start unimpeded. It is possible that the controversial antimicrobial effects of vinegar contributes to the remedial effects afforded by sub-neutral pH gels by reducing the local load of infectious agents and thus the risk for primary colonization [Emili, H et al., 1974; Larghi, OP et al., 1975; Fasanella, RM, 1991; Karapinar, M and Gonul, SA, 1992; Nishikawa, Y et al., 1993; Rund, CR, 1996; Entani, E et al., 1997]. However, infection is not a significant problem in the typical household burn addressed as initial and tested target of this invention.

Formulations according to the present invention have demonstrated efficacy treating a variety of accidental and planned partial skin thickness first and second degree, as well as a number of small area third degree burns. In addition, the compositions ofthe invention have shown effectiveness in the treatment of sunburns in adult volunteers. In a blind study, test subjects treated consecutively with different gel formulations that did or did not contain the active ingredient and were otherwise indistinguishable. These experiments firmly established the effectiveness of compositions of the invention.

Specifically, the effects ofthe compositions of the present invention include: rapid local analgesia without the numbing of local anesthetics, prevention/rapid reduction of hyperernia, prevention or reduction of local edema, prevention of blister formation, prevention of wound infection, acceleration ofwound healing, prevention of depigmentation; prevention of delayed hyperalgesia; and an absence of scarring and keloid formation.

Most of these effects are newly discovered and disclosed here. However, a possible antibacterial effect of vinegar and other apolar weak acids is controversial in the literature and, if present, could conceivably contribute to the excellent wound healing characteristics observed following early, transient application of the invention on surface burn injuries.

The compositions of the invention contain a carefully controlled acid concentration, typically derived from a weak organic acid as defined by claim 1 compatible with human skin. In a preferred embodiment, the composition comprises 0.5 to 2% acetic acid or vinegar and 2 to 8% citric acid. In a most preferred embodiment, the composition comprises 1% acetic acid or vinegar and 5% citric acid.

The composition has a pH in the range of about 4.0 to about 4.5. The selection of the pH for the composition is dependent upon the formulation used and the ability of the other components in the formulation to tolerate the acidic pH. Preferably, the pH range for effectiveness is generally about 4.0 to about 4.5, with a preferred pH ranging from 4.1 to 4.4, and a more preferred pH ranging from 4.15 to 4.25 and a most preferred pH of approximately 4.2. The gel formulations based upon Carbopol™, a pH of about 4.2 is preferred as it makes the formulation of the gel easier.

The composition is formulated as a gel to provide for longer lasting coverage of the affected areas of the skin. The gel is a water based gel using a suitable gelling agent.

The compositions of the present invention is formulated as a water soluble gel which provides sustained concentrations of weak acid. The gel formulation, for example, can utilize Carbopol™ as the gelling agent. Carbopol™ is a common gelling agent in foods, cosmetics, prescription and OTC drugs, is highly hydrophilic and rapidly removed under running water. This allows for ease of re-application to prolong the analgesic effects of the composition. While Carbopol™ is one example of a gelling agent, it will be apparent to those skilled in the art that other known gelling agents may be utilized in addition to or in place of Carbopol™, provided they are effective to form a gel in the desired pH range and do not affect the usefulness of the organic acid.

The composition may also include herb extracts deemed attractive for their purported soothing and cosmetic effects. For example, a calibrated extract of Marigold and Aloe vera may improve odor and provide added soothing properties. Herb extractions can be performed as short term (20 min, 25° C) vinegar or 5% acetic acid infusions of dried, powdered herbs at pH 2.7±0.3 (adjusted to approximately pH 4.2).

The compositions of the present invention are of particular utility for small household and kitchen burns as well as sunburns, i.e. over 95% of burn/scald accidents in North America.

Delivery of the composition of the present invention as quickly as possible after the injury is an important element for its full effectiveness. Delayed application of the formulation shows slowly lessening effectiveness, but repeated administration hours after the insult has been tested successfully after sunburns to reduce pain, hyperemia and blister formation, and produce relief from thermal hypersensitivity.

Depending on wound depth and area, single or repeated acute application ofthe compositions of the invention leads to a high degree of functional recovery within minutes and excellent wound healing course without blistering and scarring even in subjects that tend to develop keloid. In subjects tested on one or more occasions to date, the composition ofthe invention has not, so far, allowed development of depigmentation and delayed hyperalgesia.

While for most small burns, a single application of the composition of the invention provide effective relief, for optimum healing it is preferred that the composition be applied 2-4 times over the first two hours after the burn trauma.

The present invention has demonstrated, for the first time, that topical application of H⁺ (hydrogen ion) through an acidic formulation eliminates or, at least, significantly reduces the normal symptoms (hyperesthesia, blistering, etc.) and subsequent complications (infection, scarring, etc.) of first, second and small area third degree thermal injuries. H⁺ application also accelerates the healing process.

In order to prolong the beneficial effect ofthe composition within the burned area of local thermal injury, the composition is preferably provided as a gel formulation, for example using Carbopol™ 940 NF, a commercially available, commonly used thickening agent in food, cosmetic and various health care products. In addition, since herbs and/or their extracts have been traditionally used for their soothing effects, Aloe Vera, Marigold, their extracts and similar substances nay be utilized in the acid gel. However, the possible effects or effectiveness of the latter is independent of and cannot affect the activities of the weak acid.

The embodiments ofthis invention may be formulated into a kit format, comprising a composition for treating burns comprising a therapeutically effective amount of one or more weak organic acids selected from the group comprising acetic acid, vinegar, and citric acid, in a suitable carrier, wherein the pH of the composition ranges from 4.0 to 4.5, packaged along with wrapping or bandage materials, and instructions for use. Further substances that assist in occluding the damaged tissue from the environment may be included in the kit, along with other substances that assist in the treatment of the burned skin. Applicators that assist in delivering the composition to damaged tissue may also be included in the kit.

Preferred embodiments of the invention will now be described in the following examples. It is to be appreciated that the invention is not limited to the specific examples which are merely illustrative of the preferred embodiments.

### PREPARATION OF ACID GELS

Acetic acid and citric acid was each made to 5% (wt/v) in distilled H2O from glacial acetic acid (99.8 %) and solid citric acid monohydrate (analytical grade), respectively. Mixtures comprising 4 or 5% citric acid and 1% acetic acid were also made from citric acid monohydrate and commercial, food-grade vinegar containing 5% acetic acid. Powdered dry marigold and Aloe Vera were then added to these acid solutions or to vinegar to 0.15 % and 0.075 %, respectively. This step is optional and omission of the herbs does not affect the potency of the final products. The mixtures were incubated and stirred at room temperature for 20 min, and cellulose filtered, followed by the addition of EDTA to 0.1 %. Subsequently, solid Carbopol™ 940 NF or 980 NF was applied slowly to 1.8 % while being stirred at high magnetic setting until the suspension lost granularity. 10N NaOH was then slowly stirred in to adjust pH of the mixtures to 4.2 for their gelling. For examples, for every 100 mL of the vinegar and 5% acetic acid gels, 2.8 mL of the alkaline solution was required, whereas for the 5% citric acid gel, 4.3 mL was required. The actual requirement for 10N NaOH may vary slightly, depending on the specific pH parameter of the distilled water used. Finally, excess air was removed from the gel suspensions by low speed (2000 rpm) centrifugation at room temperature for 5 min.

As a control to the acid gels, a neutral (pH 7.0) gel was similarly made from distilled H₂O-(herb)-EDTA and Carbopol™ 940 NF or 980 NF. Since Carbopol™-mediated gelling is much more efficient at near neutral pH, significantly less Carbopol™ was used for the neutral gel. In addition, a neutral gel was also made from 5% acetic acid titrated with 10N NaOH.

These formulations are summarized below:

| **Preparation I: Vinegar Formulation** | |
|---|---|
| Ingredient | Amount |
| Vinegar | 100 mL |
| EDTA | 0.1 g |
| Carbopol™ | 1.8 g |
| 10N NaOH | 2.8 mL |

| **Preparation II: 5% Acetic Acid Formulation** | |
|---|---|
| Ingredient | Amount |
| glacial acetic acid (99.8%) | 5.01 mL |
| Distilled H₂O | 94.99 mL |
| EDTA | 0.1 g |
| Carbopol™ | 1.8 g |
| 10N NaOH | 2.8 mL |

| **Preparation III: 5% Citric Acid Formulation** | |
|---|---|
| Ingredient | Amount |
| Citric acid monohydrate | 5.5 g |
| Distilled H₂O | to 100 mL |
| EDTA | 0.1 g |
| Carbopol™ | 1.8 g |
| 10N NaOH | 4.3 mL |

| **Preparation IV: 5% Citric Acid + Vinegar Formulation** | |
|---|---|
| Ingredient | Amount |
| Citric acid monohydrate | 5.5 g |
| Distilled H₂O | to 80 mL |
| Vinegar | 20 mL |
| EDTA | 0.1 g |
| Carbopol™ | 1.8 g |
| 10N NaOH | 5.2 mL |

| **Preparation V: 4% Citric Acid + Vinegar Formulation** | |
|---|---|
| Ingredient | Amount |
| Citric acid monohydrate | 4.4 g |
| Distilled H₂O | to 80 mL |
| Vinegar | 20 mL |
| EDTA | 0.1 g |
| Carbopol™ | 1.8 g |
| 10N NaOH | 4.5 mL |

| **Preparation VI: Distilled H**_{**2**}**O Formulation at pH 7.0** | |
|---|---|
| Ingredient | Amount |
| Distilled H₂O | 600 mL |
| EDTA | 0.1 g |
| Carbopol™ | 1.8 g |
| 10N NaOH | 1.9 mL |

| **Preparation VII: Neutral Formulation (pH 7.0) from 5% Acetic Acid and NaOH** | |
|---|---|
| Ingredient | Amount |
| glacial acetic acid (99.8%) | 5.01 mL |
| Distilled H₂O | 94.99 mL |
| EDTA | 0.1 g |
| Carbopol™ | 1.8 g |
| 10N NaOH | 11.0 mL |

Application of the active ingredients in a clean gel form is particularly preferred as it adds benefits intrinsic to a controlled, tested and quality assured product. In particular, a gel provides sustained delivery of active ingredient(s) for a reasonable time as well as a shield from microbial attack. A gel formulation and packaging in UV resistant containers reduces loss ofvolatiles [De, VM et al., 1987] and allows addition ofingredients to augment its healing properties, smell, cosmetic appeal, transportability and shelf life.

The effectiveness of the burn therapy of the present invention is enhanced by quick application, preferably right after the thermal insult. A period of less than 5 minutes between insult and application is optimal, 10-30 minutes is acceptable. The effectiveness is further enhanced by 2 to 4 repeat applications over the first 1 to 2 hours, especially for relief of pain associated with the thermal injury. Beyond that time frame, further applications of the composition generally do not provide enhanced therapeutic effect. In sunburns where burn trauma is acquired over a prolonged period of time, the effect of the composition is less immediate, and in severe cases may require repeated applications over the first several hours of treatment.

The compositions of the invention are applied liberally over the affected skin area directly and quickly. Positive results are essentially immediate. The pain relief is dramatic, quick and lasting after single or repeated application within the first 2 hrs of trauma. If not complete, the composition of the invention can be freely re-applied. This is especially true for the gel which rinses off instantly with water if required.

The immediate perception following application of the composition of the invention is a cooling, soothing effect, followed by receding wound pain. It has been typical that burn victims will pursue whatever activity preceded the accident within some 5-10 minutes and essentially forget the insult. In superficial bums the typical post-burn hyperalgesia disappears within 10-15 minutes, in deeper injuries this process takes a day, but does not impede the ability to have, for example, a normal shower, re-applying if subjective or objective symptoms (edema, hyperemia) linger, as was the case with several small, but full thickness skin thermotraumas successfully treated with the composition of the invention.

The absence of scarring has been observed with all test applications (including the above third degree burns), as has the absence ofblistering. This applies as well to superficial, intermediate and deep partial skin thickness bums.

Initial impressions pointed to occasional depigmentation [Kahn, AM and Cohen, MJ, 1996], until it became clear that affected skin areas had light to absent pigmentation that contrasted with the tanned surrounding. These lighter areas later tanned in concert with their surrounding skin without demarcation. This observation indicates effective replacement ofthe injured skin area with healthy new skin tissue rather then collagen-rich, melanocyte depleted scar tissue.

Scar formation is a serious difficulty in burn management, yet the understanding of functional tissue elements and tissue injury responses that drive scar development are in their infancy [Swann, DA et al., 1988; Carr, CJ, 1992; Robson, MC et al., 1992; Garg, HG et al., 1993; Gibbons, M et al., 1994; Garg, HG et al., 1995; Ghahary, A et al., 1995; Harland, DL et al., 1997]. Analyses of the mechanisms that prevent scar formation following treatment with the composition ofthe invention may shed new light and delineate possible new therapeutic strategies in this central area of burn management.

The effectiveness of the composition of the invention in cases of sunburn, including one serious case with burn to some 40% of body surface area has been demonstrated. In sunburns the thermal and radiation injury accumulates relatively slowly and evenly over the affected areas. The composition of the invention was applied within one hour after leaving the sun, i.e. much later in the burn response process than after acute heat burns. The effect of the composition of the invention was dramatic, but much slower. The impressive hyperemia receded within an hour of freely applying and re-applying the composition of the invention, interestingly leaving out several small areas unintentionally not covered by the gel. This implies strictly local mechanisms of action. By the end of the day, this victim had a lukewarm shower to remove some dry gel residue - the gel dissolves instantly in water - and a thin layer was applied for the night, including areas previously uncovered but still showing local hyperalgesia and hyperemia. There was no sign of sunburn next day, the skin was comfortable to touch. The skin showed no hypersensitivity to re-exposure to sun. However, sunblocks were used more freely. There was development of natural tan, suggesting restoration of normal skin response to sunlight.

### EXAMPLES

### Example 1: In vivo testing of the acid gel in subjects with thermal skin injury

Forty seven adult volunteers were recruited into these studies. These subjects had undergone accidental or voluntary partial skin thickness surface bums. They were treated with the compositions prepared as above at pH 4.2, or aqueous solutions of 5% acetic acid at pH 2.7. Some of the test subjects underwent testing on repeated occasions. This included the two near-full thickness (third degree) small area bums tested: both were successfully treated. The main measured response elements were: speed and extent of local analgesia, development and extent of hyperemia, development and extent of local edema, development and extent of blister formation, development and extent of wound infection, time course of wound healing, development and extent of depigmentation; development and extent of delayed hyperalgesia; development and extent of scarring and keloid formation.

The initial formulations were vinegar-based, but subsequently formulations based on typically 5% acetic or citric acid were developed and compared to neutral pH (water-based) control gels. All formulations with weak acid of the invention performed in a similar fashion, employing a blinded subsequence (N-of-one) protocol.

The application of a liquid composition was reasonably effective to provide pain relief It is preferred that the composition is provided in a carrier medium to maintain the contact of the composition with the skin for extended periods. Such carrier medium may be either a physical object such as sponge, gauze, tissue or towel to contain the liquid in the place of injury or may be a more viscous composition such as a gel or lotion.

**Examples 2 - 14** are all descriptions of accidental burns and scalds. No infectious complications were observed following application of any of the gel formulations, or liquid applications of vinegar or citric acid. While interpersonal variations are considerable, there was no doubt in the observers - one of whom is a physician - that the combination of 5% citric acid plus 1% vinegar or acetic acid was the most effective formulation with lasting effect, while citric acid gels were the least effective. Gel application rapidly after injury had the fastest, most impressive effects. These impressions were confirmed in 48 non-accidental burns (see Table 1 and Example 15) during an ongoing pre-clinical fact finding and pilot study involving adult volunteers.

### Example 2 (Reference)

A white female (ID # 2) 46 years of age burned her dorsal hand with a bowl of hot soup. Within one minute of the trauma she immerged her hand in a bowl of vinegar (@ 5% acetic acid content). Pain was relieved within 20 seconds, pain reappeared gradually upon withdrawal from vinegar, and disappeared upon re-immersion. After about 5 minutes she removed hand from vinegar as the pain was gone, she resumed normal activity. No blisters and scars developed, she took a shower that evening and next morning without signs of hyperalgesia.

### Example 3 (Reference)

A white waiter, 22 years of age (ID #22) was accidentally burned by a co-worker on his upper arm with a hot fajita frying pan. The burn was severe, 7 cm long and deep. Within less than 5 minutes, a clean, liberally vinegar soaked towel was applied to this wound, a second application was done 10 minutes later. Pain relief was achieved within less than 30 seconds of first application. Within 15 minutes post trauma, the waiter resumed normal work activity in the restaurant's lunch rush period. Two hours later the towel wrap, no dry, was removed and he was sent to a nearby walk-in clinic. There, a second degree, probably IIb, was diagnosed, a sterile bandage and analgesics were offered, the latter declined by the patient. The lack of blistering was noted. An invitation for wound revisions over the next several days was declined. Wound healing proceeded without complications, there was no hyperalgesia and the wound was fully closed 4 weeks post trauma.

### Example 4 (Reference)

A white female waitress, 21 years of age (ID # 24) scalded her entire dorsal hand area with fresh brewed coffee. Within 2 minutes we applied liquid vinegar followed by application of a liberally vinegar-soaked towel. Pain relief was complete within less than 30 seconds, and the person resumed her waiting duties unencumbered within 10 minutes of the accident. No blisters or scars developed.

### Example 5 (Reference)

A white male waiter, 26 years of age (ID #25) burned his ventral thumb region with a hot fajita frying pan. Within 2 minutes, a linen towel was applied, liberally soaked with vinegar. Pain relief was complete within 20 seconds, the linen towel was removed within 10 minutes and normal work activities resumed. No blisters or scars developed.

### Example 6 (Reference)

A white male, 50 years of age (ID # 42) accidentally burned the ventral hand region with a roasting pan, causing severe pain, local analgesics were applied with little relief. The trauma was immerged in a bowl of vinegar 40 minutes post trauma. Pain relief was complete within 1 minute, but it required 35-40 minutes before pain relief was stable and the victim could remove his hand from the bowl. Incipient blistering did not proceed, there was no hyperalgesia.

### Example 7 (Reference)

A black male cook, 31 years of age (ID #41) accidentally burned his dorsal hand region with boiling oil. Liquid vinegar was applied within 3 minutes. Pain relief was complete within 30 seconds. Vinegar gel (5%) was applied 2 minutes later, and pain relief was sustained. Normal work activity was resumed within 20 minutes post trauma. No blisters or scars developed. This victim had a very similar accident 5 months previously and was treated in a hospital emergency ward with several follow-up visits. He could not resume work until 12 days later.

### Example 8 (Invention)

A white male waiter, 32 years of age (ID #39) accidentally burned the ventral region of 3 fingers on a hot plate. We applied Acetic Acid Gel (5%) within 5 minutes. Pain relief was complete within 30 seconds. Gel was reapplied 3 times over the next 10 minutes, normal work routine was resumed within 15 minutes. No blisters or scars developed.

### Example 9 (Invention)

A white waitress, 48 years of age (ID # 44) accidentally scalded a large dorso-ventral forearm region with hot soup. We applied 5% Citric Acid Gel to the burn injury within 3 minutes. Pain relief was complete within 45 seconds. The gel was reapplied 3 times over the next 15 minutes. Normal work activity was resumed within 25 minutes. No blisters or scars developed.

### Example 10 (Invention)

An Asian waitress, 35 years of age (ID #45) accidentally covered the dorsal hand region with boiling oil. We applied 5% Citric Acid & 1% Vinegar Gel within 4 minutes. Pain relief started immediately and was complete within less than 30 seconds. The gel was reapplied twice over the next 10 minutes and then removed. No pain redeveloped and normal work activity was resumed within 20 minutes. No blisters or scars evolved.

### Example 11 (Invention)

A white waiter, 34 yeas of age (ID #46) was accidentally burned with a frying pan in the dorsal forearm region. We applied 5% Citric Acid & 1% Vinegar Gel within 1 minute. Pain relief was complete within 15 seconds. The gel was reapplied once and removed within 10 minutes, when normal work activity was resumed. No pain redeveloped. No blisters or scars evolved.

### Example 12 (Invention)

A white male, 32 years of age (ID # 47) was accidentally burned with a frying pan on the ventral forearm. 5% Citric Acid & 1% Vinegar Gel was applied within 5 minutes. Pain relief began immediately and was complete within 20 seconds. The gel was reapplied twice over the next 15 minutes and then removed. No pain redeveloped. No blisters or scars evolved.

### Example 13 (Invention)

A white waitress, 29 years of age (ID #48) was accidentaliy burned with a frying pan on the ventral forearm. We applied 5% Citric Acid & 1% Vinegar Gel within 1 minute. Pain relief was complete within 15 seconds. The gel was reapplied once and removed 10 minutes post trauma. No pain redeveloped and normal work activity resumed. No blisters or scars evolved.

### Example 14 (Invention)

A white male, 46 years of age (ID #6) was burned with a glue gun on the dorsal forearm. We applied neutral 'Water Gel' within one minute. There was no pain relief, but the gel seemed to cool the injury somewhat. By 2 hours post trauma 5% Citric Acid & 1% Vinegar Gel became available and was applied. Pain relief was complete within 20 minutes. A blister had developed by this time, but blister formation did not proceed and actually receded over the next following hours. The 5% Citric Acid & 1% Vinegar Gel was reapplied 4 times within that period. The wound is presently healing without complications.

### Example 15: Series of controlled burn tests on human volunteers (Invention)

Controlled, physician-observed tests on human, adult volunteers: a localized, 2.5 cm2 thermotrauma was produced on the dorso-lateral forearm through 5-7" contact with a heated (300 ± 50° C) metallic probe. This produces a 2nd degree burn trauma with blistering and intense pain. Untreated it will develop into a second degree exacerbated (category IIb-type) trauma in 50-60% of applications. Up to four, but usually 2 bum traumas were applied per subject and occasion. The primary test endpoint was pain relief, the secondary endpoint was presence/absence of blistering, the tertiary endpoint was presence absence of hyperalgesia (undue pain) following exposure to warm water (at a temperature chosen by the subject for normal shower) 12-14 hrs after the test insult. Test subjects were physician observed for 1 hr post trauma and several times (4-8 times) over a 2-4 week period thereafter.

For ethical reasons, the effective formula was provided to all volunteers by the end of this period, usually after 60 minutes. Several different formulations were tested successively and repeatedly on the same burn, to provide comparative data. These tests were only occasionally blinded. In the 48 patients (see Table 1) of these non-accidental burns provided, only results of the initial treatment is indicated. Acceleration of wound healing was not an endpoint of this study, but the very rapid re-gain of function and absence of 1.) a single secondary infection, 2.) a single incident of posttraumatic wound exacerbation was a uniform finding.

### Example 16: Critical experiment was performed on human volunteers. (Invention)

A ∼10cm² forearm skin area was overlaid with either acetic acid/citric acid- or water gel for 45 seconds and the gel then removed with a dry cotton swab. The standard heat trauma was then applied to the same areas. There was protection from pain in the area previously overlaid with acetic acid/citric acid gel, but not the water (neutral) gel, where pain relief was rapid following application of a composition of the instant invention. While pain relief was consistently observed in the traumata pre-treated with the acetic/citric acid gel formulation ofthe instant invention, there was limited, but definitive blistering, and no accelerated healing in several tests.

The present invention provides an attractive, affordable, chemically stable and easily transportable choice for the majority of burn accidents.

Although various preferred embodiments of the present invention have been described herein in detail, it will be appreciated by those skilled in the art, that variations may be made thereto without departing from the spirit of the invention or the scope of the appended claims.

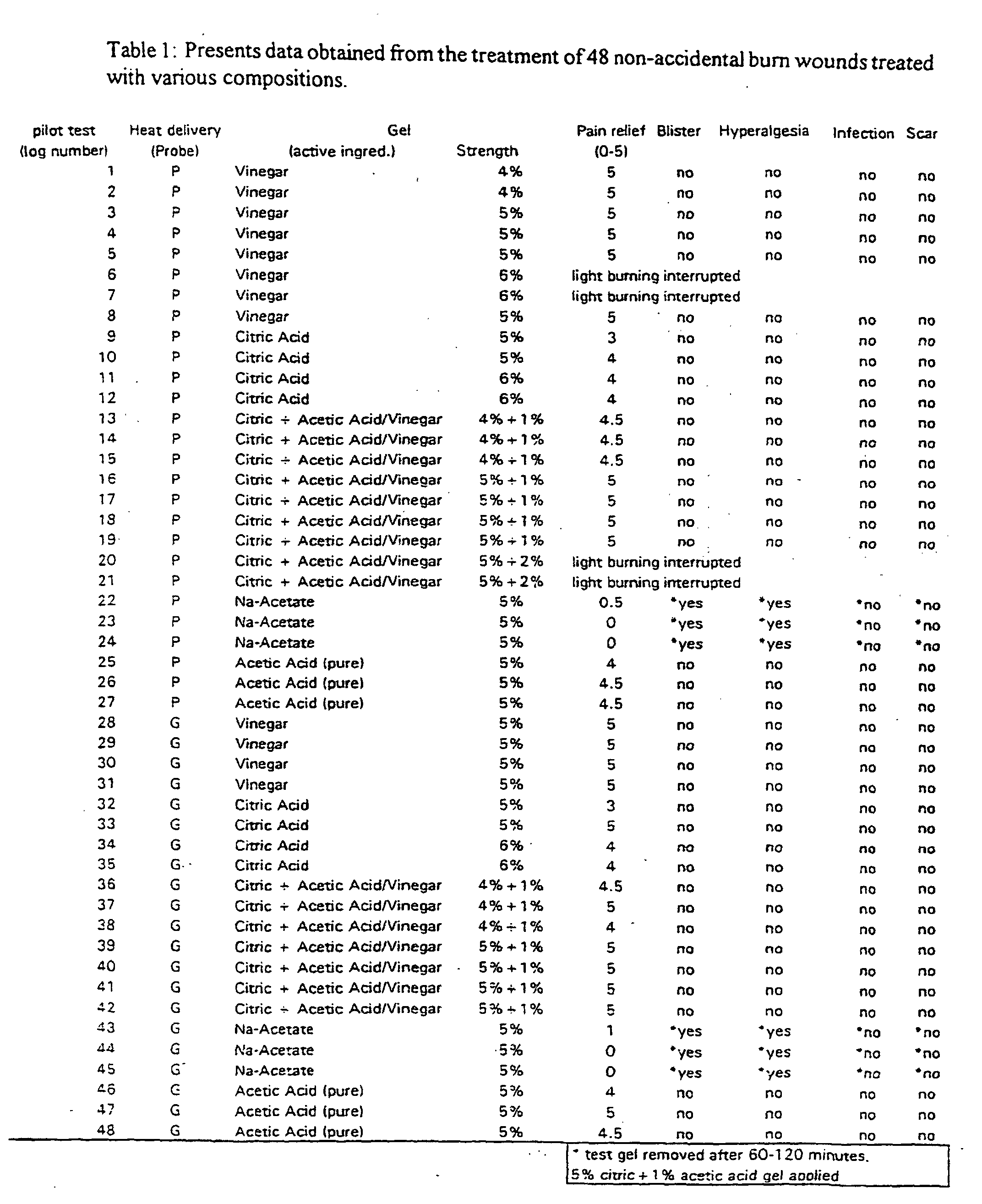

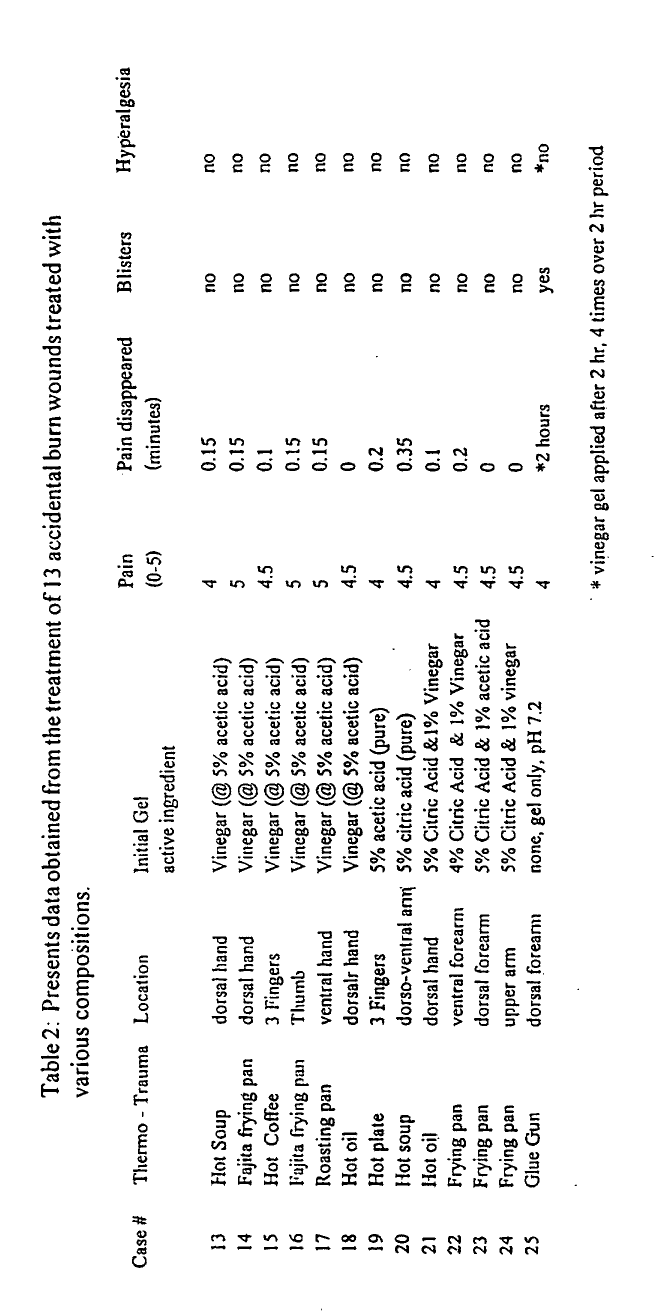

## Claims

1. A composition for treating burns comprising a therapeutically effective amount of one or more weak organic acids selected from the group: acetic acid, vinegar, and citric acid, in a carrier which includes a gelling agent, wherein the pH of the composition ranges from 4.0 to 4.5.

2. A composition as in claim 1, wherein the composition comprises 0.5 to 2% acetic acid or vinegar and 2 to 8% citric acid.

3. A composition according to claim 1 comprising 1% acetic acid or vinegar and 5% citric acid.

4. A composition according to claim 1, wherein the pH ranges from 4.1 to 4.4.

5. A composition according to claim 4 wherein the pH ranges from 4.15 to 4.25.

6. A composition according to claim 4 in which the pH is approximately 4.2.

7. A composition according to claim 1, wherein the composition includes one or more agents selected from sun blocking agents, skin moisturizing agents and herb extracts.

8. Use of the composition of claim 1 in the manufacture of a medicament for the treatment of burns.

9. Use of the composition of claim 4 in the manufacture of a medicament for the treatment of burns.

10. Use as in claim 8 wherein the composition includes one or more agents selected from sun blocking agents, skin moisturizing agents, and herb extract.

11. Use as in claim 10 wherein the composition includes a Marigold and/or aloe vera extract.

12. Use as in claim 8 wherein said medicament provides one or more or the following benefits: analgesia, prevention or reduction of hyperaemia, prevention or reduction of local edema, prevention of blister formation, prevention of wound infection, acceleration of wound healing, prevention of depigmentation, prevention of delayed hyperalgesia and absence of scarring and keloid formation.

## Patentansprüche

1. Zusammensetzung zur Behandlung von Verbrennungen, umfassend eine therapeutisch wirksame Menge einer oder mehrerer schwacher organischer Säuren, die ausgewählt sind aus der Gruppe: Essigsäure, Essig und Citronensäure, in einem Träger, der ein Gelierungsmittel einschließt, wobei der pH der Zusammensetzung im Bereich von 4,0 bis 4,5 liegt.

2. Zusammensetzung nach Anspruch 1, in der die Zusammensetzung 0,5 bis 2 % Essigsäure oder Essig und 2 bis 8 % Citronensäure umfasst.

3. Zusammensetzung nach Anspruch 1, umfassend 1 % Essigsäure oder Essig und 5 % Citronensäure.

4. Zusammensetzung nach Anspruch 1, in der der pH im Bereich von 4,1 bis 4,4 liegt.

5. Zusammensetzung nach Anspruch 4, in der der pH im Bereich von 4,15 bis 4,25 liegt.

6. Zusammensetzung nach Anspruch 4, in der der pH etwa 4,2 beträgt.

7. Zusammensetzung nach Anspruch 1, in der die Zusammensetzung ein oder mehrere Mittel einschließt, die ausgewählt sind aus Sonnenabschirmungsmitteln, Hautbefeuchtungsmitteln und Kräuter-Extrakten.

8. Verwendung der Zusammensetzung nach Anspruch 1 bei der Herstellung eines Medikaments zur Behandlung von Verbrennungen.

9. Verwendung der Zusammensetzung nach Anspruch 4 bei der Herstellung eines Medikaments zur Behandlung von Verbrennungen.

10. Verwendung wie in Anspruch 8, bei der die Zusammensetzung ein oder mehrere Mittel einschließt, die ausgewählt sind aus Sonnenabschirmungsmitteln, Hautbefeuchtungsmitteln und Kräuter-Extrakten.

11. Verwendung wie in Anspruch 10, bei der die Zusammensetzung einen Ringeiblumen- und/oder Aloe Vera-Extrakt einschließt.

12. Verwendung wie in Anspruch 8, bei der das Medikament einen oder mehrere der folgenden Vorteile bereitstellt: Analgesie, Verhütung oder Verringerung von Hyperämie, Verhütung oder Verringerung von lokalem Ödem, Verhütung einer Blasenbildung, Verhütung einer Wundinfektion, Beschleunigung der Wundheilung, Verhütung einer Depigmentierung, Verhütung einer verzögerten Hyperalgesie und Abwesenheit von Narbenund Keloid-Bildung.

## Revendications

1. Composition de traitement des brûlures comprenant une quantité thérapeutiquement efficace d'un ou plusieurs acides organiques faibles choisis dans le groupe formé d'acide acétique, vinaigre, et acide citrique, dans un véhicule qui comprend un agent gélifiant, dans laquelle le pH de la composition s'échelonne de 4.0 à 4,5.

2. Composition selon la revendication 1, dans laquelle la composition comprend de 0,5 à 2% d'acide acétique ou de vinaigre et de 2 à 8% d'acide citrique.

3. Composition selon la revendication 1 comprenant 1% d'acide acétique ou de vinaigre et 5% d'acide citrique.

4. Composition selon la revendication 1, dans laquelle le pH s'échelonne de 4,1 à 4,4.

5. Composition selon la revendication 4, dans laquelle le pH s'échelonne de 4,15 à 4,25.

6. Composition selon la revendication 4, dans laquelle le pH est d'environ 4,2.

7. Composition selon la revendication 1, dans laquelle la composition comprend un ou plusieurs agents choisis parmi les agents faisant effet d'écran solaire, les agents hydratants de la peau, et les extraits d'herbes.

8. Utilisation de la composition selon la revendication 1, dans la fabrication d'un médicament pour le traitement des brûlures.

9. Utilisation de la composition selon la revendication 4 dans la fabrication d'un médicament pour le traitement des brûlures.

10. Utilisation selon la revendication 8, dans laquelle la composition comprend un ou plusieurs agents choisis parmi les agents faisant effet d'écran solaire, les agents hydratants de la peau, et les extraits d'herbe.

11. Utilisation selon la revendication 10, dans laquelle la composition comprend un extrait de souci et/ou d'aloès vera.

12. Utilisation selon la revendication 8, dans laquelle ledit médicament procure un ou plusieurs des avantages suivants : une action analgésique, la prévention ou réduction de l'hyperémie, la prévention ou réduction de l'cedème local, la prévention de la formation d'ampoules, la prévention de l'infection des plaies, l'accélération de la cicatrisation, la prévention de la dépigmentation, la prévention de l'hyperalgie différée et l'absence de formation de chéloïdes et de cicatrices marquantes.
